(19) Europäisches Patentamt
European Patent Office
Office européen des brevets

(11) EP 0 709 456 A2

(12) **EUROPÄISCHE PATENTANMELDUNG**

(43) Veröffentlichungstag:
**01.05.1996 Patentblatt 1996/18**

(51) Int. Cl.[6]: **C12N 9/04**, G01N 33/50

(21) Anmeldenummer: **95116621.4**

(22) Anmeldetag: **21.10.1995**

(84) Benannte Vertragsstaaten:
**AT CH DE DK ES FR GB IE IT LI**

(30) Priorität: **26.10.1994 DE 4438206**

(71) Anmelder: **BOEHRINGER MANNHEIM GMBH**
**D-68298 Mannheim (DE)**

(72) Erfinder:
- **Ambrosius, Dorothee, Dr.**
  **D-81477 München (DE)**
- **Kaluza, Klaus, Dr.**
  **D-83670 Bad Heilbrunn (DE)**
- **Gross, Stefan**
  **D-80337 München (DE)**

(54) **Mikrobielle Cholesterin-Dehydrogenase, Verfahren zu deren Herstellung und Verwendung**

(57) Sauerstoffunabhängiges cholesterinumsetzendes Enzym erhältlich aus Actinomyceten oder Basidiomyceten, welches künstliche Elektronenakzeptoren verwendet sowie ein Verfahren und Reagenz zur Bestimmung von (Gesamt-) Cholesterin unter Verwendung des Enzyms.



Printed by Rank Xerox (UK) Business Services
2.11.3/3.4

**Beschreibung**

Die Erfindung betrifft ein sauerstoffunabhängiges cholesterinumsetzendes Enzym, ein Verfahren zu dessen Gewinnung aus bestimmten Mikroorganismen sowie die Verwendung des Enzyms zur Bestimmung von Cholesterin.

Die quantitative Bestimmung von Cholesterin im Blut mit Hilfe enzymatischer Tests ist seit langem eine bewährte Methode in der klinischen Chemie (Flegg 1973, Richmond 1973). Als Enzym wird dabei in der Regel eine Cholesterinoxidase eingesetzt, die die Oxidation von Cholesterin (5-Cholesten-3-β-ol) zu 4-Cholesten-3-on und $H_2O_2$ katalysiert.

$$\text{Cholesterin} + O_2 \xrightarrow{\text{Chol-OD}} \text{Cholestenon} + H_2O_2$$

Als Elektronenakzeptor dient in diesem Fall Sauerstoff. Die Bereitstellung dieses Enzyms im industriellen Maßstab aus Mikroorganismen der Klassen Schizophyllum, Streptoverticillium, Brevibacterium, Nocardia Rhodococcus oder Streptomyces (Noma & Nakayama 1976, EP 0560 983; Liu et al. 1980, Ishizaki 1989, Halpern 1981, Aihara et al. 1986, Fujishiro et al. 1990, EP 0452 112) ist etabliert.

Die Sauerstoffabhängigkeit der Cholesterin-Oxidase ist jedoch ein wesentlicher Nachteil und der Hauptgrund für Testungenauigkeiten. Sie bereitet bei der Testkalibrierung häufig Probleme, da die Abhängigkeit vom Sauerstoffpartialdruck sowohl in einer Höhenabhängigkeit als auch in einer Temperaturabhängigkeit des Tests resultiert.

Die quantitative Cholesterinbestimmung aus Blut mit Hilfe von Cholesterin-Oxidasen über eine gekoppelte Farbreaktion ist darüber hinaus wegen des entstehenden $H_2O_2$ störanfällig: $H_2O_2$ wird aufgrund seiner hohen Reaktionsfähigkeit, beispielsweise mit Bilirubin oder Arzneimitteln, dem Gleichgewicht der Reaktion entzogen.

In DE 2649749 ist ein sauerstoffunabhängiges cholesterinumsetzendes Enzym, d. h. eine NAD- bzw. NADP-abhängige Cholesterin-Dehydrogenase, welche aus einem anaeroben Mikroorganismus (Eubacterium sp.) oder aus Lebergewebe von Warmblütern erhältlich ist, beschrieben. Der wesentliche Nachteil des in dieser Anmeldung beschriebenen Enzyms besteht darin, daß auch die Isolierung des Enzyms unter Inertgas-Atmosphäre erfolgen muß. Eine Hochdimensionierung des Verfahrens und Bereitstellung des besagten Enzyms im größeren Maßstab ist nicht durchführbar. Darüber hinaus benötigt das Enzym als Cofaktor NAD(P), so daß ein zusätzlicher Reaktionsschritt (enzymatisch oder chemisch) zur Farbbildung notwendig ist. Dies hat in der Regel eine Erhöhung der Kosten und eine Steigerung der Störanfälligkeit zur Folge. Entsprechende NAD(P)-unabhängige Dehydrogenasen sind bisher lediglich für andere Substrate, wie beispielsweise Glucose oder Glycerin, bekannt (Duine 1991, Ameyama et al. 1985, Ameyama 1982. EP 0354 441, Ameyama et al. 1981, EP 0 120 440).

Der Erfindung liegt somit die Aufgabe zugrunde, ein cholesterinumsetzendes Enzym zur Verfügung zu stellen, durch das die oben genannten Nachteile überwunden werden.

Die Aufgabe wird gelöst durch ein cholesterinumsetzendes Enzym, welches künstliche Elektronenakzeptoren (Mediatoren) verwendet und aus bestimmten Mikroorganismen erhältlich ist. Als artifizielle Elektronenakzeptoren (Mediatoren) kommen insbesondere unsubstituierte oder substituierte Benzochinone, Indophenol- und Nitrosoanilinderivate in Betracht. Unter den substituierten Benzochinon- bzw. Nitrosoanilinverbindungen sind solche bevorzugt, die nicht-elektronenziehende Reste am aromatischen Grundgerüst tragen, wie beispielsweise niedrige Alkylgruppen. Alkylgruppen mit bis zu 10 C-Atomen haben sich hier als besonders geeignet erwiesen.

Insbesondere haben sich als artifizielle Elektronenakzeptoren Methyl-1,4-Benzochinon (MBQ), p-Benzochinon (PBQ) und N,N-Dimethyl-4-nitrosoanilin (NA), aber auch 2,6-Dichlorphenolindophenol (DCIP) und N,N-bis-2(Hydroxyethyl)-p-Nitrosoanilin als geeignet erwiesen.

Der wesentliche Vorteil des erfindungsgemäßen Enzyms ist, daß es die Elektronen direkt anstatt auf Sauerstoff oder NAD(P) auf künstliche Elektronenakzeptoren überträgt, was direkt zur Signalausbeute benutzt werden kann. Im Gegensatz zu den klassischen Nachweismethoden mit Hilfe von Cholesterin-Oxidase ist das Meßsystem unabhängig vom Sauerstoffpartialdruck. Somit wird eine Sauerstoffstörung vermieden.

Das erfindungsgemäße Enzym kommt insbesondere in Mikroorganismen, wie Actinomyceten oder Basidiomycen-Arten, vor. Insbesondere konnte eine entsprechende Cholesterin-Dehydrogenase (Chol-DH) aus bestimmten Rhodococcus- und Streptomyctes-Stämmen (Rhodococcus spec. BMTU 3899 DSM 9444, Rhodococcus erythropolis DSM 743, Streptomycetes hygroskopicus DSM 40771) isoliert werden. Als Pilzstämme haben sich im wesentlichen bestimmte Basidiomyces-Arten (Flammulina velutipes DSM 1658, Coprinus comatus DSM 1746, Trametes versicolor BMTU 3107, DSM 9443) als Quelle für die erfindungsgemäße Cholesterin-Dehydrogenase als geeignet erwiesen.

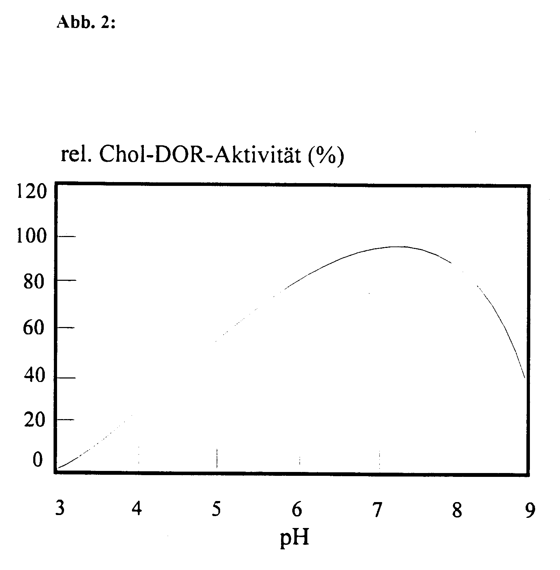

Das erfindungsgemäße Enzym zeichnet sich darüber hinaus durch einen optimalen pH-Wert-Bereich von 6 bis 10 und eine optimale Temperatur zwischen 25 °C und 40 °C aus. Außerdem weist das Enzym eine hohe Temperaturstabilität auf: Ohne Zusatz eines Stabilisators zeigt das Enzym nach ca. 30 Minuten bei 60 °C noch mindestens 90 %, in der Regel noch 98 - 100 % der Ausgangsaktivität (Abb. 1). Die größte pH-Stabilität zeigt die erfindungsgemäße Chol-DH im Bereich von pH 7,0 bis 8,0 (Abb.2).

Ferner ist das Enzym durch ein apparentes Molekulargewicht von ungefähr 55.000 Da (SDS-PAGE) charakterisiert. Der isoelektrische Punkt der Chol-DH liegt bei ungefähr pH 8,6 (Amphilie PAG plate pH 3,5 - 9,5; 2,5 Stunden, 1500 V, 30 mA und 30 W).

Ein weiterer Gegenstand der Erfindung ist ein Verfahren zur Gewinnung der Cholesterin-Dehydrogenase, wobei geeignete Bakterien- bzw. Pilzstämme, die eine Cholesterin-Dehydrogenase enthalten, zunächst in einem geeigneten Wachstumsmedium kultiviert werden. Ein geeignetes Wachstumsmedium beruht auf Hefeextraktbasis und weist neben den sonst üblichen Nährsalzen und Spurenelementen Cholesterin in Konzentrationen von ca. 0,5 bis 5 g/l auf. Insbesondere bei den Anzuchten der Bakterienstämme konnte mit Tryptic Soy (Difco, Art.Nr. 0370) eine Verdoppelung der Expression erzielt werden. Darüber hinaus wurde durch Zusatz von Detergentien (0,1 - 5 %; (w/v)), wie z. B. Tween 80, eine weitere Expressionssteigerung um den Faktor 4 erreicht. Ebenso hat sich hier Taurocholat als geeignet erwiesen.

Anschließend erfolgt die Isolierung des Enzyms aus dem Kulturüberstand mittels fraktionierter Ammoniumsulfatfällung, Dialyse und einem oder mehreren chromatographischen Reinigungsschritten. Insbesondere hat sich für die chromatographische Reinigung der Chol-DH als vorteilhaft erwiesen, wenn zunächst eine Hydroxyapatit und anschließend eine Phenylsepharose- und Superdex-200-Säule verwendet wird. Die Isolierung des Enzyms aus den Biomassen erfolgt analog, wobei zusätzlich ein geeigneter Aufschlußpuffer und Maßnahmen für den Zellaufschluß eingesetzt werden. Die Zellen können dabei chemisch, enzymatisch (z. B. Lysozym-Behandlung) oder mechanisch (Druck, Zellmühle, Glasperlen etc.) aufgeschlossen werden. Vorteilhafterweise wird die Enzymfraktion nach dem Zellaufschluß und der Extraktion des Enzyms mit organischem Lösungsmittel gewaschen.

Die Chol-DH konnte auf diese Weise mit einer Reinheit von mindestens 99 % (beispielsweise SDS-PAGE oder RP-HPLC; Methoden sind dem Fachmann bekannt), einer spezifischen Aktivität von 2 bis 5 U/mg und einer Ausbeute von ca. 10 % (bezogen auf das Dialysat nach Ammoniumsulfat-Fällung, d. h. nach Abtrennung höher molekularer Komponenten wie Lipide usw.) erreicht werden.

Ein weiterer Gegenstand der Erfindung betrifft die Verwendung des erfindungsgemäßen Enzyms zur Bestimmung von Gesamt-Cholesterin (frei oder gebunden) in biologischen Proben, wie sie in der klinischen Chemie Anwendung findet. Dabei wird die Chol-DH-Aktivität entweder über die Bildung von Cholestenon oder über die Reduktion des zugesetzten Redoxmediators ermittelt. Die Bildung von Cholestenon wird beispielsweise mit Hilfe einer HPLC-Methode oder die Reduktion des Redoxmediators photometrisch oder elektrochemisch verfolgt.

Ein entsprechendes Reagenz zur Bestimmung von Gesamt-Cholesterin (frei oder gebunden) besteht üblicherweise aus Cholesterin-Esterase, sauerstoffunabhängiger Cholesterin-Dehydrogenase, Puffer, gegebenenfalls einem Stabilisierungsmittel oder/und einem oberflächenaktiven Mittel sowie einem System zur Bestimmung von Cholestenon bzw. des reduzierten Mediators. Entsprechende Systeme zur Bestimmung von Cholestenon sind dem Fachmann bekannt. Die Bestimmung des reduzierten Mediators kann direkt erfolgen, und zwar durch Bestimmung der Absorptionsabnahme bei einer Wellenlänge - je nach verwendetem Mediator - zwischen 300 und 700 nm. Bei Verwendung von DCIP, PBQ oder MBQ wird bei einer Wellenlänge von ungefähr 600 nm die Absorptionsabnahme spektroskopisch verfolgt.

Die quantitative Zusammensetzung eines derartigen Reagenzes beträgt zweckmäßig 0,02 bis 4 U/ml Cholesterin-Esterase, 0,02 bis 4 U/ml sauerstoffunabhängige Chol-DH und 0,01 bis 10 mM eines Elektronenakzeptors, vorzugsweise 0,05 bis 1 mM.

Falls das Reagenz ein oberflächenaktives Mittel, wie beispielsweise Thesit oder Cholat enthält, so liegt dessen Konzentration zweckmäßig zwischen 0,1 und 10 % (w/v).

Mit dem erfindungsgemäßen Verfahren und Reagenz läßt sich eine sehr rasche und vollständige Bestimmung von gebundenem und freiem Cholesterin, insbesondere auch in biologischen Proben wie Serum, Blut, Plasma und dergleichen durchführen. Ein besonderer Vorteil liegt darin, daß keine zusätzlichen Meßreaktionen angekoppelt werden müssen und die Bestimmung direkt mit den weitverbreiteten einfachen Photometern durchgeführt werden kann.

<u>Abbildung 1:</u> Stabilität der Chol-DH (1,0 U/ml) unter Temperaturbelastung; 30 min Inkubation in Wasserbädern der entsprechenden Temperatur; Angabe der Chol-DH-Aktivität relativ zur Ausgangsaktivität

<u>Abbildung 2:</u> Stabilität der Chol-DH (1,0 U/ml) bei Belastung unter verschiedenen pH-Bedingungen bei 25 °C für 3 h; Angabe der Chol-DH-Aktivität relativ zur Ausgangsaktivität;
verwendete Puffer:pH 3,0 - 6,0: 0,2 M $Na_2HPO_4$/Citrat-Puffer
pH 7,0 - 9,0: 0,2 M $K_2HPO_4$/KOH-Puffer

**LITERATUR**

1. Flegg, H.M. (1973) An investigation of the determination of serum cholesterol by an enzymatic method. Ann. Clin. Biochem. 10: 79

2. Richmond, W. (1973) Preparation and properties of a cholesterol oxidase from Nocardia sp. and its application to the enzymatic assay of total cholesterol in serum. Clin. Chem. 19: 1350 - 1356

3. Noma, A. & Nakayama, K. (1976) Comparative studies on cholesterol oxidases from different sources. Clin. Chim. Acta 73: 487 - 496

4. Liu, W., Cheng, C. & Su, Y. (1980) Isolation and identification of a cholesterol oxidase-producing bacterium. Proc. Natl. Sci. Counc. ROC 4: 433 - 437

5. Ishizaki, T., Hirayama, N., Shinkawa, H., Nimi, O. & Murooka, Y. (1989) Nucleotide sequence of the gene for cholesterol oxidase from a Streptomyces sp. J. Bacteriol. 171: 596-601

6. Halpern, M. G. (1981) Cholesterol oxidase from bacteria. pp. 3 - 22; In: Industrial Enzymes from Microbial Sources, Noyes Data Corporation

7. Aihara, H., Watanabe, K. & Nakamura, R. (1986) Characterization of production of cholesterol oxidases in three Rhodococcus strains. J. Appl. Bacteriol. 61: 269 - 274

8. Fujishiro, K., Ohta, T., Hasegawa, M., Yamaguchi, K., Mizukami, T. & Uwajima, T. (1990) Isolation and identification of the gene of cholesterol oxidase from Brevibacterium sterolicum ATCC 21387, a widely used enzyme in clinical analyses. Biochem. Biophys. Research Communications 172: 721 - 727

9. Fujishiro, K. & Uwajima, T. (1991) Cholesterol oxidase. EP 0 452 112 A1

10. Duine, J. A. (1991) Quinoproteins: Enzymes containing the quinonoid cofactor pyrroloquinoline quinone, topaquinone or tryptophan-tryptophan quinone. Eur. J. Biochem. 200: 271 - 284

11. Ameyama, M., Shinagawa, E., Matsushita, K. & Adachi, O. (1985) Solubilization, purification and properties of membrane-bound glycerol dehydrogenase from Gluconobacter industrius. Agric. Biol. Chem. 49: 1001 - 1010

12. Ameyama, M. (1982) Enzymatic microdetermination of D-glucose, D-fructose, D-gluconate, 2-keto-D-gluconate, aldehyde, and alcohol with membrane-bound dehydrogenases. Methods Enzymol. 28: 20 - 29

13. Ameyama, M. (1990) NAD(P)-unabhängige Glycerindehydrogenase, Verfahren zu deren Herstellung sowie deren Verwendung zur Bestimmung von Glycerin und Triglyceriden. EP 0 120 440 B1

14. Ameyama, M., Shinagawa, E., Matsushita, K. & Adachi, O. (1981) D-glucose dehydrogenase of Gluconobacter suboxydans: Solubilization, purification and characterization. Agric. Biol. chem. 45: 851-861

15. Hoenes, J. (1990) Verfahren zu kolorimetrischen Bestimmung eines Analyten mittels enzymatischer Oxidation. EP 0 354 441 A2

Die folgenden Beispiele erläutern die Erfindung weiter.

**Beispiel 1:** Züchtung von Mikroorganismen, die eine Cholesterin-Dehydrogenase enthalten

**1.1 Bakterien**

Die Cholesterin-Dehydrogenase konnte beispielsweise aus folgenden Organismen isoliert werden:
Rhodococcus erythropolis DSM 743
Rhodococcus spec. BMTU 3899, DSM 9444
Streptomyces hygroskopicus DSM 40771
Die Kultivierung dieser Bakterienstämme erfolgte nach bekannten Methoden: Von den Bakterienstämmen wurden Reinigungsausstriche auf Standard I-Nähragarplatten (Merck Art.-Nr. 7881) angelegt. Ausgehend von diesen Reinigungsausstrichen wurden Vorkulturen in Standard I-Nährbouillon (Merck Art.-Nr. 7882) angelegt. Dazu wurden 50 ml Nährbouillon in 250 ml-Erlenmeyerkolben mit einer einzelnen Kultur des Reinigungsausstrichs angeimpft und 24 h bei 28 °C im Brutraum auf einem Horizontalschüttler mit 150 Upm inkubiert. Ausgehend von diesen Vorkulturen wurden Hauptkulturen in obigem Wachstumsmedium angelegt. Dazu wurden 800 ml des Wachstumsmediums in 2,0 l-Erlenmeyerkolben mit 15 ml Vorkultur angeimpft und 60 h bei 28 °C im Brutraum auf einem Horizontalschüttler mit 150 Upm inkubiert.

Folgendes Wachstumsmedium wurde verwendet; alle Medienbestandteile wurden in Wasser (dest.) gelöst.

| Wachstumsmedium: | |
|---|---|
| $Na_2HPO_4 \cdot 2\,H_2O$ | 5,25 g/l |
| $KH_2PO_4$ | 1,50 g/l |
| NaCl | 0,10 g/l |
| $MgSO_4 \cdot 7\,H_2O$ | 0,10 g/l |
| Hefeextrakt | 0,50 g/l |
| $NH_4Cl$ | 1,00 g/l |
| Spurenelemente (-Lösung) | 1,00 ml/l |
| Cholesterin-Lösung | 100,00 ml/l |
| Spurenelemente (-Lösung): | |
| $MnCl_2 \cdot 4\,H_2O$ | 0,50 mg/l |
| $ZnSO_4 \cdot 7\,H_2O$ | 1,00 mg/l |
| $CuSO_4 \cdot 5\,H_2O$ | 0,50 mg/l |
| $CoCl_2 \cdot 6\,H_2O$ | 0,50 mg/l |
| $NiCl_2 \cdot 6\,H_2O$ | 0,50 mg/l |
| $Na_2MoO_4 \cdot 2\,H_2O$ | 0,05 mg/l |
| $FeCl_3 \cdot 6\,H_2O$ | 1,50 mg/l |
| Cholesterin-Suspension: | |
| Cholesterin | 10,0 g/l |
| Tween-80 | 7,5 g/l |

Eine enzymatische Aktivität konnte sowohl in den Kulturüberständen als auch in den Biomassen gemessen werden.

**1.2 Pilze**

Beispiele für Chol-DH aus Basidiomyces-Arten

Beispiel 1.2.1

20 ml einer Nährlösung mit 0,1 % Hefeextrakt (Difco), 0,2 % Ammoniumtartrat, 0,1 % $KH_2PO_4$, 0,05 % $MgSO_4$, 0,05 % KCl und 1 % Glukose in einem 100 ml Erlenmeyer-Kolben wurden mit Flammulina velutipes BMTU 3683, DSM 1658 beimpft. Diese Vorkultur wurde unter Schütteln 6 Tage bei 28 °C inkubiert.

Als Hauptkultur wurden 500 ml einer Nährlösung mit 0,5 % Hefeextrakt (Difco), 1 % Malzextrakt, 0,001 % $FeCl_3$, 1 % Glukose, mit einem pH von 6,0 in einem 2 l Erlenmeyer-Kolben mit oben beschriebener Kultur versehen und weitere 6 Tage auf einem Schüttler inkubiert.

Nach der Kultivierung wurden die Zellen durch Filtration abgetrennt.

In dem Kulturüberstand konnte eine Enzymaktivität von 3,5 mU/ml nachgewiesen werden.

**Photometrische Methode:**

Als Redoxmediator zur Bestimmung der Chol-DH-Aktivität aus Basidiomyceten wurde 2,6-Dichlorphenolindophenol (DCIP) eingesetzt. Die Reduktion von DCIP wurde über Absorptionsabnahme bei 600 nm gemessen.

Verwendete Reagenzien:

1. 0,1 M MOPS/KOH, pH 7,5
2. 2 mM DCIP in Puffer 1
3. 5 % NaCholat/5 % Na Desoxycholat in Puffer 1
4. Katalase (BM Cat. No. 0156 744)
5. 15 mM Cholesterin gelöst in Puffer 3

| | Leerwert | Probe |
|---|---|---|
| Puffer 1 | 729 µl | 729 µl |
| DCIP (2) | 66 µl | 66 µl |
| Katalase (4) | 5 µl | 5 µl |
| Lösung (3) | 50 µl | 50 µl |
| Enzymprobe (ca. 10 mU/ml) | 50 µl | 50 µl |
| mischen und warten bis Vorreaktion abgelaufen ist | | |
| Cholesterin (5) | | 100 µl |
| Lösung (3) | 100 µl | |
| mischen und bei $A_{600}$ messen | | |

Die Chol-DH-Aktivität wird wie folgt bestimmt:
Eine Unit ist die Enzymaktivität, die 1 µmol Cholesterin pro Minute unter den Testbedingungen (37 °C, pH 7,0) zu Cholestenon oxidiert und dabei gleichzeitig 1 µmol $DCIP_{ox}$ reduziert.

$$\text{Volumenaktivität } \frac{1,0}{16,13 \times 0,05} \text{ Verdünnungsfaktor der Probe } \Delta A/\text{min [U/ml Probe]}$$

Beispiel 1.2.2

100 ml einer Vorkultur von Flammulina velutipes BMTU 3683 wurden, wie in Beispiel 1.2.1 beschrieben, kultiviert und zu 1,7 l einer Nährlösung von 0,3 % Hefeextrakt (Difco), 1 % Polypepton M66 (Merck), 0,3 % $KH_2PO_4$, 0,1 % $MgSO_4$, 1 % Glukose in einer 5 l Steilbrustflasche gegeben. Die Kultur wurde 6 Tage bei 28 °C unter Schütteln inkubiert.

Im Kulturüberstand wurde eine Aktivität von 2,7 mU/ml bei einer spezifischen Aktivität von 20 mU/mg Protein gemessen. Der Überstand wurde mit 0,1 % Tween 80 versetzt und mit einer Diafiltration (10 kD Membran, Filtron Ultrasette™) konzentriert. Das dabei mit 20 mM Tris/HCl pH 7,5 gewaschene Filtrat wurde auf eine 5 cm DEA-Membran (Sartorius) aufgetragen und mit 0,3 M NaCl (Eluat 1) bzw. 0,6 M NaCl (Eluat 2) eluiert. Eluat 1 enthielt eine Gesamtaktivität von 0,7 U mit einer spezifischen Aktivität von 0,24 U/mg Protein, Eluat 2 0,3 U mit einer spez. Aktivität von 1,03 U/mg.

Beispiel 1.2.3

20 ml einer Nährlösung mit 0,3 % Hefeextrakt, 1 % Polypepton (Merck), 0,3 % $KH_2PO_4$, 0,1 % $MgSO_4$, 1 % Stärke wurden mit Trametes versicolor BMTU 3107, DSM 9443 beimpft.

Nach 5 Tagen Wachstum bei 28 °C wurde die Kultur in 500 ml desselben Mediums überführt und weitere 5 Tage in einem 2 l Erlenmeyer-Kolben geschüttelt. Im Kulturfiltrat wurde eine Aktivität von 1,5 mU/ml gemessen.

Beispiel 1.2.4

Coprinus comatus BMTU 3680, DSM 1476 wurde, wie in Beispiel 1.2.4 beschrieben, kultiviert. Im Kulturfiltrat wurde eine Aktivität von 0,4 mU/ml gemessen.

**Beispiel 2: Isolierung der Cholesterin-Dehydrogenase**

**a) Isolierung aus dem Überstand**

Der Kulturüberstand einer Fermentation von Rhodococcus spec. (DSM 9444) wurde gegen 50 mM Kaliumphosphatpuffer pH 7,0 diafiltriert (30.000 open-channel Membran). Die Chol-DH wurde mit Hilfe von Ammoniumsulfat aus dem Diafiltrat gefüllt. Dabei wurden in einem ersten Schritt (1,2 M Ammoniumsulfat) Fremdproteine entfernt und die Chol-DH dann quantitativ bei 2,4 M Ammoniumsulfat gefällt. Das Präzipitat wurde in 10 mM Kaliumphosphatpuffer pH 7,0 gelöst und gegen diesen Puffer dialysiert. Die Reinigung der Chol-DH erfolgte über Chromatographie an Hydroxylapatit (pH 6,8), Phenylsepharose und Superdex-200 (pH 7,0).

Bei der Trennung an Hydroxylapatit wurde zunächst die Säule (V = 40 ml) mit Puffer A (10 mM $KH_2PO_4$/KOH; pH 6,8 plus 0,3 mM $CaCl_2$) bei 10 °C und einer Flußrate von 40 ml/h äquilibriert. Für die Trennung wurde ein Gradient von je 200 ml Puffer A und Puffer B (350 mM $KH_2PO_4$/KOH; pH 6,8 plus 10 mM $CaCl_2$) benutzt. Insgesamt wurden 200 mg Protein auf die Säule aufgetragen, wobei die Probe eine Proteinkonzentration von 5 mg/ml besaß.

Als weiter Reinigungsschritt eignet sich die Phenylsepharose (10 ml Säule), die in Startpuffer (50 mM $KH_2PO_4$/KOH; pH 7,0,1 M Ammoniumsulfat) äquilibriert wurde. Alle Schritte erfolgten bei 10 °C und einer Flußrate von einem Säulenvolumen pro Stunde. Die Säule wurde mit 20 mg Protein beladen, wobei die Proteinkonzentration der Probe 2 mg/ml betrug. Der Gradient für die Elution wurde mit Hilfe von 50 ml Startpuffer und 50 ml Puffer B (10 mM $KH_2PO_4$/KOH; pH 7,0; 0,1 % Thesit) eingestellt.

Eine zusätzliche Abtrennung von Fremdprotein wurde mit Hilfe einer Gelpermeotionschromatographie an Superdex-S200 erzielt. Die Säule wurde bei RT mit 200 mM $KH_2PO_4$/KOH; pH 7,0; 0,1 % Thesit bei einer Flußrate von 1 ml/min äquilibriert. Nach Auftrag des Probenvolumens von 1 ml (≙ 5 mg Protein) erfolgte die Trennung mit obigem Puffer.

Über das beschriebene Reinigungsschema wurde ein Enzymmuster mit einer Reinheit von > 99 % SDS-PAGE (oder RP-HPLC) und einer spezifischen Aktivität von 2,4 U/mg (bezogen auf Aktivitätstest in Beispiel 3a); Proteinbestimmung nach Pierce, BCA-Assay mit RSA-Standard) bei einer Ausbeute von 9 % (bezogen auf das Dialysat nach Ammoniumsulfat-Fällung; Ermittlung der Chol-DH-Aktivität wie in Beispiel 3a) beschrieben) erhalten.

**b) Isolierung aus den Biomassen**

Als Standard-Puffer für alle Zellaufschlüsse wurden 50 mM Kaliumphosphatpuffer pH 7,0 verwendet (Aufschlußpuffer).

Gaulin-Press:

4 g tiefgefrorene Biomasse (FG) wurden in 40 ml Aufschlußpuffer mit 1 mg/ml Lysozym aufgetaut und 30 min inkubiert. Der Aufschluß erfolgte bei 1000 bar. Die beim Aufschluß freigesetzte DNA wurde durch Zusatz von 0,5 mg/ml DNase und 2.5 mg/ml $MgSO_4 \cdot 7\,H_2O$ und 30 min Inkubation bei 25 °C verdaut. Die Chol-DH kann aus dem Überstand nach Zentrifugation (SS 34, 19.000 Upm, 30 min, 4 °C), wie unter a) beschrieben, gereinigt werden.

Zellmühle (IMA-Disinteerator-S)

1 g gefrorene Biomasse (FG) wurde in Aufschlußpuffer mit 1 mg/ml Lysozym aufgetaut und 30 min bei 25 °C inkubiert. Vor Behandlung in der Zellmühle wurden dem Ansatz 10 ml Glasperlen (∅ 0,25 - 0,30 mm) zugesetzt. Der Aufschluß erfolgte 2 x 4 min mit 4000 Upm bei 4 °C. Nach Absetzen der Glasperlen wurde die Suspension aufgebrochener Zellen abgegossen. Die Glasperlen wurden zweimal mit Aufschlußpuffer nachgewaschen, um eine möglichst quantitative Ausbeute zu erhalten. Durch Zentrifugation (SS 34, 19.000 Upm, 30 min, 4 °C) wurden die Zelltrümmer sedimentiert. Die Chol-DH kann aus dem Überstand, wie unter a) beschrieben, gereinigt werden.

Membranextraktion

Die Sedimente der Zellen aus dem Zellmühlen- bzw. Gaulin-Press-Aufschluß oder ganze Zellen (1 g FG, tiefgefroren) wurden in 5 ml organischem Lösungsmittel (Butylacetat, n-Butanol oder Ethylacetat) und anschließend mit 10 ml Aufschlußpuffer gewaschen. Die Extraktion der Chol-DH aus der Zellhülle erfolgte dann durch Inkubation der gewaschenen Zellen in 5 ml Aufschlußpuffer mit 1 % Thesit für 30 min bei 37 °C unter Rühren im Wasserbad. Die Extraktionsansätze wurden abzentrifugient (SS 34, 19.000 Upm, 30 min, 4 °C) und die Chol-DH konnte aus dem Überstand, wie unter a) beschrieben, gereinigt werden.

**Beispiel 3: Bestimmung der Chol-DH-Aktivität**

Das Enzymmuster wurde, wie in Beispiel 2 a) beschrieben, gereinigt und anschließend seine Eigenschaft des Cholesterin-Umsatzes mit folgenden Methoden bewertet.

a) **HPLC-Methode**

Bei der Ermittlung der Chol-DH-Aktivität über eine Quantifizierung gebildeten Cholestenons wurde ein Enzymtest eingesetzt, bei dem die Enzymreaktion über einen definierten Zeitraum ablief, bevor sie durch Zugabe eines proteindenaturierenden Agens abgestoppt wurde. Danach wurde die Menge an Produkt bestimmt und als µmol pro Minute und Volumeneinheit der Enzymlösung (= Volumenaktivität) oder Gewichtseinheit des Proteins (= spezifische Aktivität) ausgedrückt.

Das abgebildete Cholestenon wurde nach Abstoppen der Chol-DH-Reaktion über eine HPLC-Analytik quantifiziert:

| | |
|---|---|
| Säule: | VYDAC 5 C 18-Säule (auf 37 °C temperiert) |
| Fließmittel: | 100 % Methanol (isokratisch) |
| Flußrate: | 1 ml/min |
| Detektion: | 210 nm → Cholesterin und Cholestenon |
| | 240 nm → Cholestenon |
| Laufdauer: | 10 min |

Es wurden folgende Reagenzien verwendet:

1. 50 mM Methyl-1,4-Benzochinon (in $H_2O$)
2. 0,4 % Cholesterin-Lsg.: 10 % 1-Propanol, 10 % Thesit in 50 mM Kaliumphosphat-Puffer pH 7,0
3. $H_2O_{bidest.}$
4. Eisessig
5. Ethylacetat

| In Eppendorf-Gefäße pipettieren: | Leerwert | Probe |
|---|---|---|
| Enzymprobe (30 - 40 mU/ml[1)]) | 100 µl | 100 µl |
| MBQ (1) | - | 20 µl |
| $H_2O$ (3) | 20 µl | - |
| Cholesterin (2) | 100 µl | 100 µl |
| mischen und bei 37 °C im Wasserbad inkubieren (10 - 60 min), anschließend abstoppen mit | | |
| Eisessig (4) | 15 µl | 15 µl |
| mischen und Zugabe von | | |
| Ethylacetat | 200 µl | 200 µl |
| Extraktion durch 30 min Schütteln auf Eppendorf-Schüttler; kurz zentrifugieren (Eppendorf-Zentrifuge) und obere Ethylacetat-Phase für HPLC-Analytik abnehmen | | |

[1)] Chol-DH-Aktivität nach Beispiel 3 a)

Die Chol-DH-Aktivität wird wie folgt bestimmt:
Mit Hilfe von Cholestenon (gelöst in Ethylacetat) wurde zunächst eine Eichgerade für die HPLC-Methode erstellt.

Eine Unit ist die Enzymaktivität, die 1 µmol Cholesterin pro Minute unter den Testbedingungen (37 °C, pH 7,0) zu Cholestenon oxidiert. Die Chol-DH-Aktivität ergibt sich dabei aus der Differenz der Cholestenon-Konzentrationen von Testansatz und Leerwert (s.o.).

$$\text{Volumenaktivität} = \frac{\Delta\ c\ (\text{Cholestenon}\ [\mu\text{mol/ml}]\ \cdot 2}{\text{Inkubationsdauer [min]}} \cdot \text{Verdünnungsfaktor der Probe [U/ml]}$$

b) **Photometrische Methode**

Mit 2,6-Dichlorphenolindophenol (DCIP) als Redoxmediator wurde ein photometrischer Chol-DH-Aktivitätstest aufgebaut:

$$\text{Cholesterin} + \text{DCIP}_{\text{ox.}} \rightarrow \text{4-Cholesten-3-on} + \text{DCIP}_{\text{red.}}$$

Die Reduktion von DCIP konnte über Absorptionsabnahme bei 600 nm gemessen werden.

Verwendete Reagenzien:

1. 50 mM $KH_2PO_4$/KOH-Puffer pH 7,0
2. 0,4 % Cholesterin-Lsg.: 10 % 1-Propanol. 10 % Thesit in 50 mM KPP pH 7,0
3. 4 mM DCIP in Puffer (1)
4. Katalase (BM Cat. No. 0156 744)
5. 10 % 1-Propanol/Thesit-Lsg. in Puffer (1)

| In Halbmikroküvetten pipettieren | Leerwert | Probe |
|---|---|---|
| Puffer (1) | 720 µl | 720 µl |
| DCIP (3) | 25 µl | 25 µl |
| Katalase | 5 µl | 5 µl |
| Enzymprobe (ca. 10 mU/ml[2]) | 100 µl | 100 µl |
| mischen und warten bis Vorreaktion abgelaufen ist | | |
| Lösung (5) | 150 µl | - |
| Cholesterin (2) | - | 150 µl |
| mischen und $A_{600}$ messen | | |

[2])Chol-DH-Aktivität nach Beispiel 3a)

Die Chol-DH-Aktivität wird wie folgt bestimmt:
Eine Unit ist die Enzymaktivität, die 1 µmol Cholesterin pro Minute unter den Testbedingungen (37 °C, pH 7,0) zu Cholestenon oxidiert und dabei gleichzeitig 1 µmol $DCIP_{ox}$ reduziert.

$$\text{Volumenaktivität} = \frac{1.0}{16,13 \cdot 0,1} \cdot \text{Verdünnungsfaktor der Probe} \cdot \Delta\ \text{A/min [U/ml Probe]}$$

c) **Elektrochemische Methode**

Die Ermittlung der Chol-DH-Aktivität konnte auch über quantitative Bestimmung von reduziertem Redoxmediator an einem elektrochemischen Meßplatz erfolgen. Als Redoxmediator wurde Methyl-1,4-benzochinon eingesetzt.

Cholesterin +

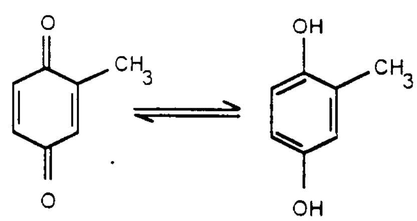

+ Cholestenon

Methyl-1,4-benzochinon (MBQ) Methylhydrochinon (MHQ)

Die Messung des gebildeten Methylhydrochinons erfolgte auf einem Dummy (Kunststoffträger mit Graphitpaste bedruckt) bei einer Meßspannung von 240 mV.

Es wurden folgende Reagenzien verwendet:

1. 50 mM Methyl-1,4-benzochinon (in $H_2O$)
2. 0,4 % Cholesterin-Lsg.: 10 % 1-Propanol, 10 % Thesit in 50 mM Kaliumphosphat-Puffer pH 7,0
3. $H_2O_{bidest.}$
4. Eisessig

| In Eppendorf-Gefäße pipettieren | Leerwert | Probe |
|---|---|---|
| Enzymprobe (0,1 - 0.2 U/ml[3)]) | 50 µl | 50 µl |
| MBQ (1) | - | 10 µl |
| $H_2O$ (3) | 10 µl | - |
| Cholesterin (2) | 50 µl | 50 µl |
| mischen und bei 37 °C im Wasserbad inkubieren (10 - 30 min). anschließend abstoppen mit | | |
| Eisessig (4) | 7 µl | 7 µl |
| Messung des Meßstroms bis zur Konstanz (ca. 2.5 min) auf Dummy | | |

[3)]Chol-DH-Aktivität nach Beispiel 3 a)

Die Chol-DH-Aktivität wird wie folgt bestimmt:
Zunächst wurde mit Methylhydrochinon als Standard unter Verwendung der Testbedingungen eine Eichgerade ermittelt. Das Δc (MHQ) berechnet sich aus der Differenz der MHQ-Konzentration des Leerwertes und der Probe.

Eine Unit ist die Enzymaktivität, die 1 µmol Cholesterin pro Minute unter den Testbedingungen (37 °C, pH 7,0) zu Cholestenon oxidiert und dabei gleichzeitig 1 µmol Methyl-1,4-benzochinon zu Methylhydrochinon reduziert.

$$\text{Volumenaktivität} = \frac{2{,}34}{t} \cdot F \cdot \Delta C\ (\text{MHQ})\ [\text{U/ml}]$$

F = Verdünnungsfaktor der Probe

**d) Variation der verwendeten Redoxmediatoren**

Neben Methyl-1,4-benzochinon sind weitere Redoxmediatoren als Elektronenakzeptoren für die Chol-DH geeignet. Die Chol-DH-Aktivität wurde über die HPLC-Methode ermittelt. In Tabelle 1 sind verschiedene Redoxmediatoren aufgelistet, die als artifizielle Elektronenakzeptoren der Chol-DH dienen können.

Tabelle 1

| Mediator | Chol-DH [%] |
|---|---|
| Methyl-1,4-Benzochinon (MBQ) | 100 |
| p-Benzochinon (PBQ) | 109 |
| Tetrachloro-p-Benzochinon | 27 |
| 2,6-Dichlorphenolindophenol (DCIP) | 52 |
| N,N-Dimethyl-4-nitrosoanilin (NA) | 95 |
| N,N-bis-2(Hydroxyethyl)p-Nitrosoanilin | 45 |

**Beispiel 4: Bestimmung der Cholesterin-Konzentration**

Mit Hilfe der folgenden Testfolge wurde gezeigt, daß die erfindungsgemäße Chol-DH zur Bestimmung der Cholesterin-Konzentration geeignet ist:

Reaktionsschema:

$$\text{Cholesterin} + \text{DCIP} \xrightarrow{\text{Chol-DH}} \text{Cholestenon} + \text{DCIP}_{\text{red.}}$$

Verwendete Reagenzien:

1. 50 mM $KH_2PO_4$/KOH-Puffer pH 7,0, 1,5 % 1-Propanol, 1,5 % Thesit
2. 4 mM DCIP in Puffer (1)
3. Katalase (BM Cat. No. 0156 744)
4. Chol-DH-Lsg.: 1 U/ml (Endkonzentration; ermittelt nach Beispiel 3 a) in 50 mM $KH_2PO_4$/KOH-Puffer pH 7,0

Ausführung:

Gemessen wird die Extinktionsabnahme bei 600 nm

Meßstrahlung: 600 nm
Testvolumen: 1,0 ml
Schichtdicke: 1,0 cm
Meßtemperatur: 37 °C

| In Halbmikroküvetten pipettieren: | |
|---|---|
| Puffer (1) | 770 µl |
| DCIP (2) | 25 µl |
| Katalase (3) | 5 µl |
| Chol-DH-Lsg. (4) | 100 µl |
| mischen, temperieren, Zugabe von | |
| Cholesterinprobe | 100 µl |
| mischen und $A_{600}$ nach 30 min messen | |

Berechnung:

$$\Delta E \cdot \frac{1,0}{16,13 \cdot 0,1} = \mu\text{mol/ml Probelösung}$$

**Beispiel 5: Chol-DH-Aktivität bei unterschiedlicher Sauerstoff-Sättigung**

Die Chol-DH-Aktivität wurde über die HPLC-Methode (Beispiel 3 a)) unter sauerstoffreduzierten, atmosphärischen und sauerstoffgesättigten Bedingungen durchgeführt. Eine Sauerstoffreduktion wurde durch Entgasen der Versuchsansätze (10 min) und anschließendes Begasen mit Stickstoff (10 min) erreicht. Darüber hinaus erfolgte die Versuchsdurchführung in einer Anaerobier-Kammer. Die Sauerstoff-Sättigung wurde mit Hilfe eines $O_2$-regenerierenden Systems durch Zusatz von $H_2O_2$ (0,01 % Endkonzentration im Testsystem) und Katalase (40 U/ml Endkonzentration im Testsystem) erreicht.

Es zeigte sich, daß eine Störung des Tests durch Sauerstoff nicht gegeben war (Tab. 2).

Tabelle 2

| $O_2$-Bedingungen | Chol-DH [U/ml] |
|---|---|
| reduziert | 13,3 |
| atmosphärisch | 13,7 |
| gesättigt | 13,7 |

**Patentansprüche**

1. Sauerstoffunabhängiges cholesterinumsetzendes Enzym erhältlich aus Mikroorganismen der Gruppierung der Actinomyceten oder Basidiomyceten, dadurch gekennzeichnet, daß es künstliche Elektronenakzeptoren verwendet.

2. Enzym nach Anspruch 1, dadurch gekennzeichnet, daß als künstliche Elektronenakzeptoren ein Benzochinon-, Indophenol- oder Nitrosoanilinderivat verwendet wird.

3. Enzym nach Anspruch 1 oder 2, dadurch gekennzeichnet, daß das Enzym Methyl-1,4-Benzochinon, p-Benzochinon, N,N-Dimethyl-4-nitrosoanilin, 2,6-Dichlorphenolindophenol oder N,N-bis-2(Hydroxyethyl)p-Nitrosoanilin als Elektronenakzeptor verwendet.

4. Enzym nach einem der Ansprüche 1 bis 3, dadurch gekennzeichnet, daß das Enzym nach ca. 30 Minuten bei 60 °C von mindestens 90 % Restaktivität aufweist, ein Maximum der Aktivität im pH-Bereich von ungefähr 7,0 bis 8,0 zeigt.

5. Enzym nach einem der Ansprüche 1 bis 4, erhältlich aus Rhodococcus spec. BMTU 3899 (DSM 9444), Rhodococcus erythropolis (DSM 743), Streptomyces hygroskopicus (DSM 40771), Flammulina velutipes (DSM 1658), Coprinus comatus (DSM 1476) oder Trametes versicolor BMTU 3107 (DSM 9443).

6. Verfahren zur Herstellung des Enzyms gemäß einem der Ansprüche 1 bis 5, dadurch gekennzeichnet, daß man zunächst eine Vorkultur auf Basis eines Hefeextraktes herstellt und anschließend das Enzym aus dem Kulturüberstand oder der Biomasse von Bakterien, gegebenenfalls durch vorherigen Zellaufschluß, durch fraktionierte Fällung und stufenweise chromatographische Reinigung isoliert.

7. Verfahren nach Anspruch 6, dadurch gekennzeichnet, daß bei den Anzuchten der Bakterienstämme Tryptic Soy als Medium verwendet wird.

8. Verfahren nach Anspruch 6 oder 7, dadurch gekennzeichnet, daß das Medium ein Detergenz oder eine Detergenzmischung enthält.

9. Verfahren nach Anspruch 8, dadurch gekennzeichnet, daß als Detergenz Tween 80 und/oder Taurocholat zugesetzt wird.

10. Verfahren nach Anspruch 8 oder 9, dadurch gekennzeichnet, daß die Detergenzkonzentration ca. 0.1 bis 5,0 % (w/v) beträgt.

11. Verfahren zur Bestimmung von Cholesterin in Gegenwart eines cholesterinumsetzenden Enzyms, dadurch gekennzeichnet, daß eine sauerstoffunabhängige Cholesterin-Dehydrogenase aus Mikroorganismen der Gruppierung der Actinomyceten oder Basidiomyceten verwendet wird und gegebenenfalls eine Cholesterin-Esterase zugegen ist.

12. Verfahren nach Anspruch 11, dadurch gekennzeichnet, daß die Cholesterin-Dehydrogenase künstliche Elektronenakzeptoren verwendet.

13. Verfahren nach Anspruch 12, dadurch gekennzeichnet, daß als Elektronenakzeptor Methyl-1,4,Benzochinon, p-Benzochinon, N,N-Dimethyl-4-nitrosoanilin, 2,6,Dichlorphenolindophenol und/oder N,N-bis-2(Hydroxyethyl)p-Nitrosoanilin verwendet wird.

**14.** Verfahren nach einem der Ansprüche 11 - 13, dadurch gekennzeichnet, daß das Enzym erhältlich ist aus Rhodococcus spec. BMTU 3899 (DSM 9444), Rhodococcus erythropolis (DSM 743), Streptomyces hygroskopicus (DSM 40771), Flammulina velutipes (DSM 1658), Coprinus comatus (DSM 1476) oder Trametes versicolor BMTU 3107 (DSM 9443).

**15.** Reagenz zur Bestimmung von Cholesterin, dadurch gekennezeichnet, daß es eine sauerstoffunabhängige Cholesterin-Dehydrogenase, Stabilisierungsmittel, einen Mediator, gegebenenfalls eine Cholesterin-Esterase, Puffer und ein Detergenz enthält.

**16.** Reagenz nach Anspruch 15, dadurch gekennzeichnet, daß das Detergenz Thesit oder Cholat ist.

**17.** Reagenz nach Anspruch 15 oder 16, dadurch gekennzeichnet, daß das Detergenz in einer Konzentration von ungefähr 0,1 bis 10 % (w/v) vorliegt.

Abb. 1:

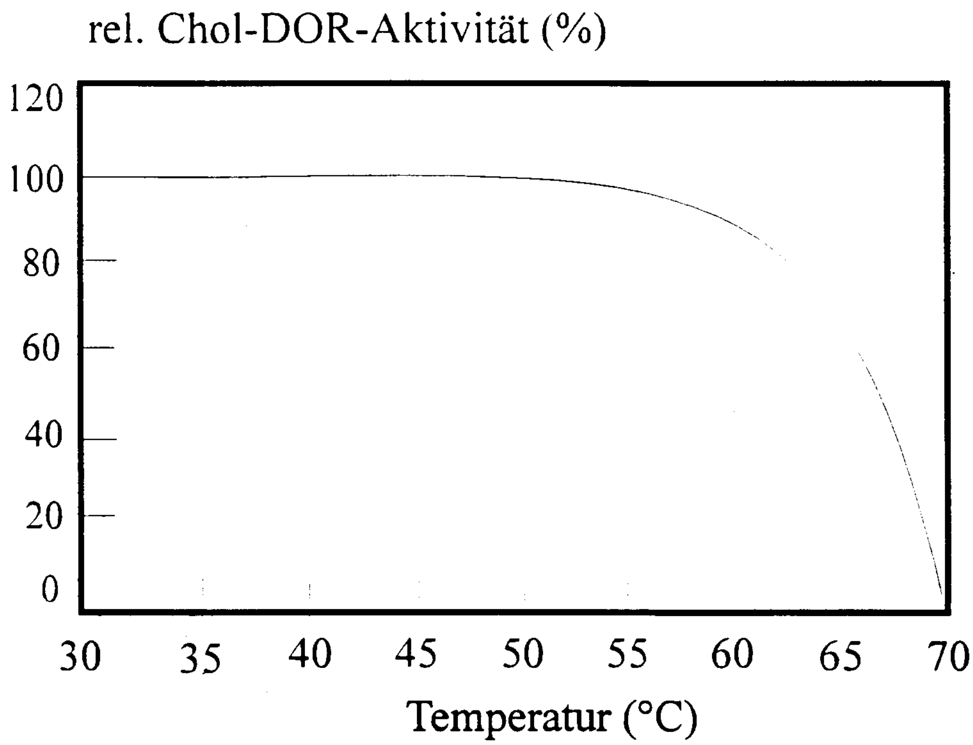
rel. Chol-DOR-Aktivität (%)
120
100
80
60
40
20
0
30
35
40
45
50
55
60
65
70
Temperatur (°C)

Abb. 2: